# EUROPÄISCHE PATENTSCHRIFT

(11) **EP 2 010 258 B1**
(45) Veröffentlichungstag und Bekanntmachung des Hinweises auf die Patenterteilung: **31.07.2013**
(21) Anmeldenummer: 07711576.4
(22) Anmeldetag: 19.02.2007
(51) Int. Cl.: A61M 15/00

(54) **TROCKENPULVER-INHALATOR**
DRY POWDER INHALER
INHALATEUR DE POUDRE SECHE

(30) Priorität: 24.02.2006 DE 102006010089
(43) Veröffentlichungstag der Anmeldung: 07.01.2009
(73) Patentinhaber: Emphasys Importadora Exportadora e Distribuidora Ltda., 18540-000 Porto Feliz SP (BR)
(72) Erfinder: ESTEVE, Viktor, Sao Paulo- SP (BR); KREIM, Achim, 64407 Fränkisch-Crumbach (DE)
(74) Vertreter: Dreiss
(86) Internationale Anmeldenummer: PCT/EP2007/001408
(87) Internationale Veröffentlichungsnummer: WO 2007/098870

(56) Entgegenhaltungen:
- EP-A- 1 350 532
- EP-A1- 0 406 893
- EP-A1- 0 491 426
- EP-A1- 0 666 085
- EP-A2- 0 950 423
- EP-A2- 1 082 971
- DE-A1- 2 704 574
- DE-A1- 19 637 125
- DE-A1- 19 704 849
- US-A- 3 906 950
- US-A- 5 372 128

## Beschreibung

Die Erfindung betrifft einen Trockenpulver-Inhalator mit einem Grundgehäuse, mit einer Kapselaufnahme für eine Kapsel mit Trockenpulver, mit wenigstens einem gegenüber dem Grundgehäuse beweglich angeordneten, nadel- oder klingenartigen Öffnungsmittel zum Öffnen der Kapsel, und mit einem Mundstück, durch welches das Trockenpulver einer geöffneten Kapsel inhalierbar ist.

Ein derartiger Trockenpulver-Inhalator ist beispielsweise aus der EP-A-1 270 034 oder der US-A-2003/0000523 bekannt.

Bei derart bekannten Inhalatoren sind separate, mit den Öffnungsmitteln fest verbundene Betätigungsmittel vorgesehen, die zum Öffnen der Kapsel in das Grundgehäuse eingedrückt werden.

Aus der US 5 372 128 A ist bekannt geworden, dass das Öffnungsmittel am Mundstück befestigt ist und dass das Mundstück gegenüber dem Grundgehäuse aus einer Normallage in eine die Kapsel öffnende Öffnungslage bewegbar ist.

Weitere, ähnliche Trockenpulver-Inhalator sind aus der DE 27 04 547 A1, EP 1 082 971 A2, DE 197 04 849 A1, EP 0 950 423 A2 und der DE 196 37 125 A1 bekannt.

Aus der EP 0491 426 A1 ist ein Trockenpulver-Inhalator bekannt geworden, bei dem das Grundgehäuse zum Einlagen der Kapsel zweiteilig aufklappbar ausgebildet ist.

Mit der vorliegenden Erfindung sollen Inhalatoren bereit gestellt werden, die einfach und bedienerfreundlich handzuhaben sind.

Zur Lösung der Aufgabe schlägt die Erfindung einen Trockenpulver-Inhalator mit den Merkmalen des Anspruchs 1 vor. Aufgrund der schwenkbaren Anordnung der Kapselaufnahme kann folglich ein einfaches und bedienerfreundliches Einführen der Kapsel in die Kapselaufnahme erfolgen. Dabei ist vorgesehen, dass die Schwenkachse, um die die Kapselaufnahme schwenkbar ist, senkrecht zur Mittellängsachse des Mundstücks oder des Grundgehäuses verläuft. Die Kapselaufnahme schwenkt dann folglich seitlich aus dem Grundgehäuse heraus. Zur schwenkbaren Anordnung der Kapselaufnahme sieht das Grundgehäuse auf seiner dem Mundstück abgewandten Seite das die Kapselaufnahme umfassende oder tragende Schwenkteil vor, das in seiner nicht ausgeschwenkten Normallage vorteilhafterweise wenigstens weitgehend bündig mit der Außenseite des Grundgehäuses abschließt. Dadurch wird erreicht, dass in der Normallage des Schwenkteils sich das Schwenkteil vorteilhafterweise in das Grundgehäuse einfügt.

Vorteilhafterweise ist ferner vorgesehen, dass die Schwenkachse, um die die Kapselaufnahme schwenkbar ist, versetzt zur Mittellängsachse des Mundstücks oder des Grundgehäuses angeordnet ist. Aufgrund dieser nicht mittigen Anordnung der Schwenkachse wird erreicht, dass schon bei geringem Aufschwenkwinkel die Kapselaufnahme gut zugänglich ist.

Um das Mundstück aus seiner Öffnungslage wieder in seine Normallage zu bringen, kann vorgesehen sein, dass zwischen dem Gehäuse und dem Mundstück wenigstens ein Federelement vorgesehen ist. Beim Bewegen beziehungsweise Drücken des Mundstücks aus der Normallage in die Öffnungslage muss folglich eine Betätigungskraft bereitgestellt werden, die größer ist als die Federkraft, mit welcher das Mundstück in der Normallage gehalten wird. Bevorzugt kann ein Federelement oder können mehrere Federelemente vorgesehen sein. Das Vorsehen mehrerer Federelemente, beispielsweise mehrerer parallel zueinander oder koaxial ineinander angeordneter Schraubenfedern, kann den Vorteil haben, dass eine gleichmäßigere Vorspannkraft bereitgestellt wird, womit einem Verkanten oder Verklemmen des Mundstücks beim Überführen aus der Öffnungslage in die Normallage entgegen gewirkt wird.

Vorteilhafterweise wird das Mundstück aus der Normallage in die Öffnungslage in axialer Richtung bezüglich der Mittellängsachse des Mundstücks beziehungsweise des Grundgehäuses bewegt. Insbesondere kann das Mundstück derart angeordnet sein, dass es beim Überführen aus der Normallage in die Öffnungslage wenigstens abschnittsweise in das Grundgehäuse eingeführt wird.

Gemäß einer vorteilhaften Ausbildung der Erfindung kann vorgesehen sein, dass als Öffnungsmittel wenigstens eine sich in Längsrichtung des Mundstücks erstreckende Nadel Verwendung findet. Die Nadel kann dabei derart angeordnet sein, dass in der Normallage des Mundstücks die Nadel nicht in die Kapselaufnahme eingreift. Beim Niederdrücken des Mundstücks gelangt dann wenigstens die Nadelspitze in die Kapselaufnahme, in welche die entsprechende Kapsel eingelegt werden kann.

Zur Führung des Mundstücks aus seiner Normallage in die Öffnungslage kann am Grundgehäuse ein im Wesentlichen hülsenartiges Zwischenteil Verwendung finden. Hierdurch wird vorteilhafterweise über die gesamte Bewegungsbahn des Mundstücks das Mundstück geführt. Das hülsenartige Zwischenteil ist dabei vorteilhafterweise so ausgebildet, dass es beim Inhalieren des Trockenpulvers das Trockenpulver von der Kapselaufnahme zum Mundstück leitet. Dazu kann das Zwischenteil auf der der Kapselaufnahme zugewandten Seite eine siebartige Struktur aufweisen, die verhindert, dass das Kapselgehäuse beim Inhalieren in das Zwischenteil beziehungsweise in das Mundstück gelangen kann. Die siebartige Struktur hält folglich die Kapsel in der Kapselaufnahme zurück.

Das Schwenkteil als solches kann dabei in Seitenansicht eine U-förmige Außenkontur aufweisen, die sich von der Vorderseite über die Unterseite bis zur Rückseite des Grundgehäuses erstrecken kann. Dies hat den Vorteil, dass ein Ausschwenken des Schwenkteils beispielsweise durch Greifen der Vorder- und der Rückseite des Schwenkteils mit zwei Fingern erfolgen kann. Insofern kann das Schwenkteil auf einfache Art und Weise aus der Normallage in die Schwenklage verschwenkt und anschließend, nach Einlegen der Kapsel, aus der Schwenklage in die Normallage zurückverschwenkt werden.

Zwischen den beiden U-Schenkeln eines derartigen Schwenkteils ist dann vorteilhafterweise die Kapselaufnahme angeordnet. Die Kapselaufnahme kann dabei einteilig mit dem Schwenkteil ausgebildet sein oder auch als separates Bauteil am Schwenkteil befestigt sein.

Die Kapselaufnahme als solche weist vorteilhafterweise eine im Wesentlichen geringfügig größer als die Kapsel ausgebildete Vertiefung auf, an die sich trichterartig hin zur Vertiefung verlaufende Flächen anschließen. Hierdurch wird erreicht, dass beim Einlegen der Kapsel in die Kapselaufnahme diese automatisch die vorgesehene Lage in der Vertiefung einnimmt. Beim Öffnen der Kapsel befindet sich dann die Kapsel in der Vertiefung; ein seitliches Ausweichen der Kapsel beim Auftreffen der Öffnungsmittel auf die Kapsel kann dadurch nicht erfolgen.

Vorzugsweise verläuft die Längsachse der Vertiefung der Kapselaufnahme schräg zu einer Mittellängsebene des Grundgehäuses. Die Längsachse der Vertiefung kann dabei beispielsweise mit der Mittellängsebene der Breitseite des Grundgehäuses einen Winkel von cirka 45° einschließen. Ein derartiger Winkel hat den Vorteil, dass die Kapsel in der aufgeschwenkten Kapselaufnahme selbst in die Vertiefung findet. Ferner kann die Kapsel nach Gebrauch des Inhalators auf einfache Art und Weise aus der Vertiefung herausgenommen werden.

Die Kapselaufnahme kann zudem durch eine im Wesentlichen kreisrunde Oberkante begrenzt werden. Dadurch kann ein definierter Abschluss beziehungsweise Anschluss an die der Kapselaufnahme zugewandte Unterseite des hülsenartigen Zwischenteils bereitgestellt werden. Im Bereich der Oberkante der Kapselaufnahme kann zudem ein Ansaugeinlass für Ansaugluft beim Inhalieren vorgesehen sein. Der Ansaugeinlass ist dabei vorteilhafterweise senkrecht zur Mittellängsachse des Gehäuses beziehungsweise des Mundstücks angeordnet.

Es ist zudem vorteilhaft, wenn die Kapselaufnahme einen Lagerraum zur Lagerung von Kapseln aufweist. Dadurch kann ein gewisser Vorrat von Kapseln im Inhalator mitgeführt werden. Dabei ist denkbar, dass der Lagerraum bei herausgeschwenkter Kapselaufnahme zum Einlegen bzw. Herausnehmen von zu lagernden bzw. gelagerten Kapseln zugänglich ist und bei eingeschwenkter Kapselaufnahme nicht zugänglich ist. Beim Transport des Inhalators mit geschlossener Kapselaufnahme sind die Kapseln im Lagerraum verliersicher untergebracht.

Weitere Einzelheiten und vorteilhafte Ausgestaltungen der Erfindung sind den Unteransprüchen und der folgenden Beschreibung zu entnehmen, in der zwei in der Zeichnung dargestellte Ausführungsbeispiele näher beschrieben und erläutert sind.

Es zeigen:
- Figur 1: eine perspektivische Ansicht eines erfindungsgemäßen Trockenpulver-Inhalators mit aufgesetztem Deckel;
- Figur 2: die Vorderansicht des Inhalators gemäß Figur 1 ohne Deckel;
- Figur 3: die Seitenansicht des Inhalators gemäß Figur 1;
- Figur 4: die Ansicht des Inhalators gemäß Figur 3 mit aufgeschwenkter Kapselaufnahme;
- Figur 5: eine perspektivische Ansicht von Figur 4;
- Figur 6: eine Draufsicht auf den Inhalator gemäß Figur 1;
- Figur 7: einen Schnitt entlang der Linie A-A gemäß Figur 6;
- Figur 8: einen Schnitt entlang der Linie D-D gemäß Figur 6;
- Figur 9: einen Schnitt entlang der Linie C-C gemäß Figur 6;
- Figur 10: den Schnitt gemäß Figur 9 mit niedergedrücktem Mundstück;
- Figur 11: eine Draufsicht auf die Unterseite des umgedrehten Inhalators; und
- Figur 12: das Schwenkteil mit abgezogener Kappe.

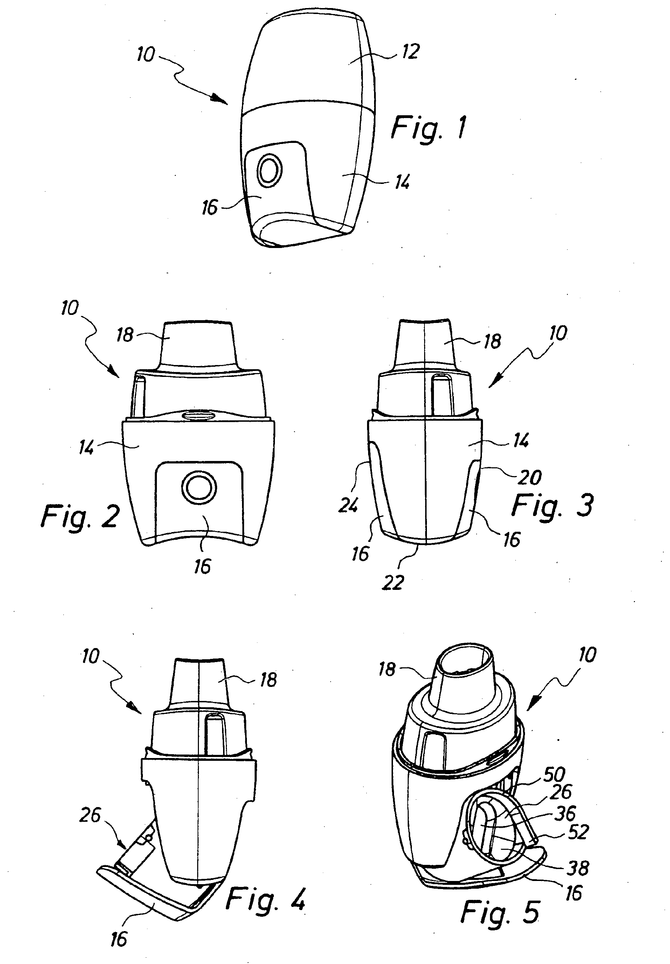

Der in der Figur 1 dargestellte Trockenpulver-Inhalator 10 umfasst einen Deckel 12, ein Grundgehäuse 14 und ein am Grundgehäuse schwenkbar angeordnetes Schwenkteil 16.

In den Figuren 2 und 3 ist der Inhalator 10 gemäß Figur 1 ohne Deckel 12 dargestellt. Sichtbar ist ein in Längsrichtung des Inhalators 10 verlaufendes, hohl ausgebildetes Mundstück 18, über das Trockenpulver einer im Inhalator vorhandenen, geöffneten Kapsel inhalierbar ist.

Wie aus insbesondere Figur 3 deutlich wird, erstreckt sich das Schwenkteil 16 von der Vorderseite 20 des Grundteils über die Unterseite 22 des Grundteils hin zur Rückseite 24 des Grundteils. In Seitenansicht ist das Schwenkteil 16 folglich U-förmig ausgebildet. In der in den Figuren 1 bis 3 dargestellten Normallage des Schwenkteils schließt dieses weitgehend bündig mit der Außenseite des Grundgehäuses 14 ab.

In den Figuren 4 und 5 ist das Schwenkteil 16 in einer aus dem Grundgehäuse 14 herausgeschwenkten Schwenklage gezeigt. In der Schwenklage ist eine am Schwenkteil 16 angeordnete Kapselaufnahme 26 zugänglich. In die Kapselaufnahme 26 ist eine Trockenpulverkapsel einlegbar. Nach dem Einlegen der Kapsel wird das Schwenkteil wieder in seine Normallage zurückgeschwenkt.

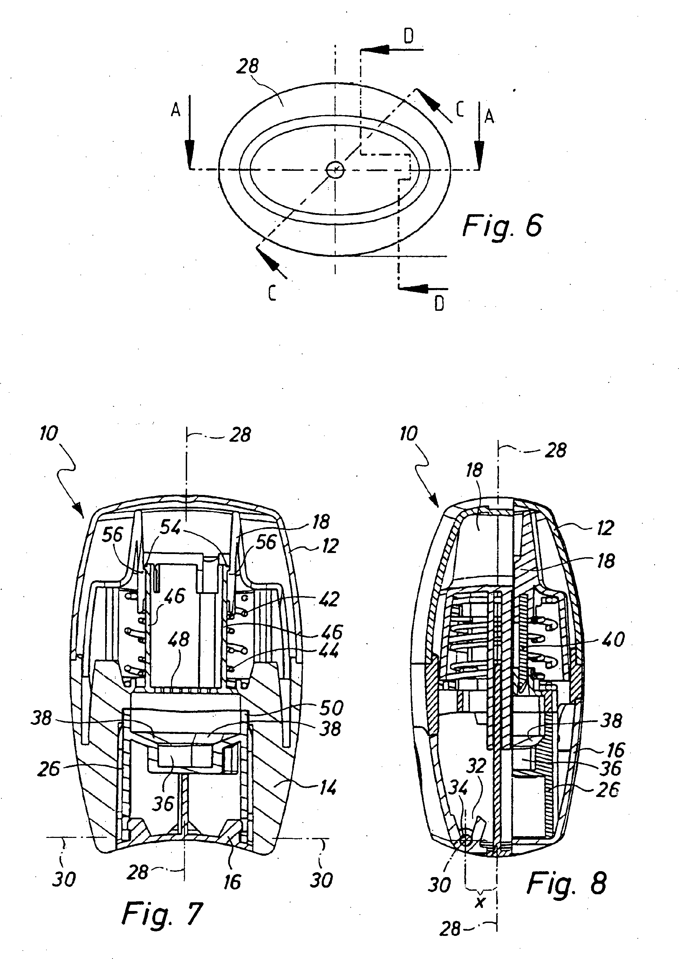

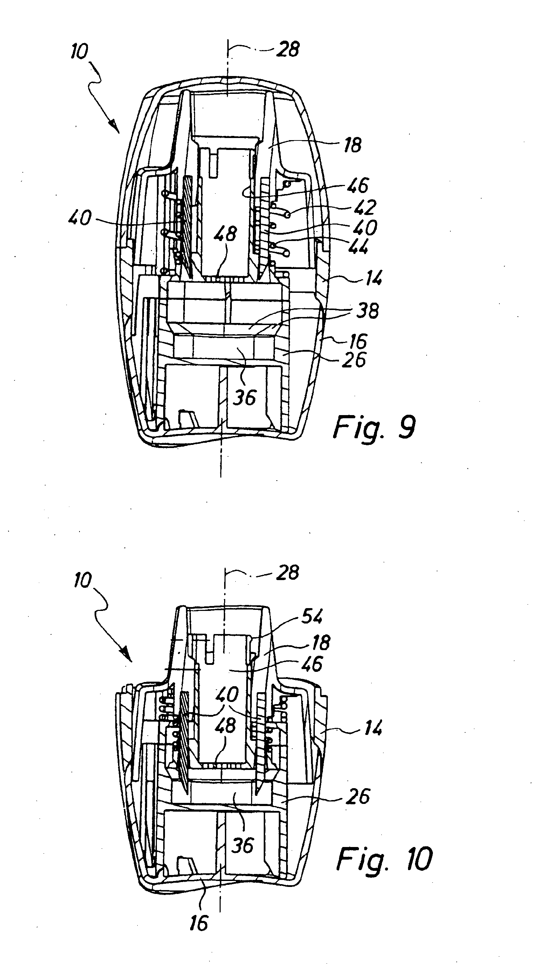

Wie insbesondere aus den Figuren 7 und 8 hervorgeht, ist das Schwenkteil 16 um eine um den Wert x versetzt zur Mittellängsachse 28 des Grundgehäuses beziehungsweise des Mundstücks angeordnete Schwenkachse 30 beabstandet angeordnet. Die Schwenkachse 30 verläuft zudem senkrecht zur Mittellängsachse 28. Das Schwenkteil 16 sieht zur schwenkbaren Anordnung V-förmig geöffnete Schwenksaussparungen 32 vor, in die in am Grundteil 14 einander gegenüber liegend und einander zugewandte Schwenkzapfen 34 eingreifen.
Insbesondere aus den Schnitten der Figuren 7, 8, 9 und 10 wird deutlich, dass die Kapselaufnahme 26, die am Schwenkteil 16 befestigt ist, eine im Wesentlichen geringfügig größer als die aufzunehmende Kapsel ausgebildete Vertiefung 36 aufweist, an die sich trichterartig hin zur Vertiefung verlaufende Flächen 38 anschließen. Die Vertiefung 36 verläuft dabei schräg zu den Mittellängsebenen der Schmalseite und Breitseite des Grundgehäuses und des Mundstücks, wobei der Schnitt A-A in Figur 6 entlang der Mittellängsebene der Breitseite des Grundgehäuses 14 beziehungsweise des Inhalators 10 verläuft. Dies wird insbesondere auch aus Figur 5 deutlich. Der in Figur 9 dargestellte Schnitt C-C gemäß Figur 6 verläuft entlang der Längsrichtung der Vertiefung.

Aus Figur 9 und 10 wird deutlich, dass am Mundstück 18 zwei Öffnungsmittel in Form von in Längsrichtung des Mundstücks verlaufende Nadeln 40 angeordnet sind. Das Mundstück 18 ist zusammen mit den Nadeln 40 entlang der Längsachse 28 nach unten zum Grundgehäuse hin niederdrückbar. Figur 9 zeigt die Normallage des Mundstücks 18 und Figur 10 die niedergedrückte Öffnungslage des Mündstücks 18. In der niedergedrückten Öffnungslage ragen die Nadeln 40 in die Kapselaufnahme 26 und zudem in die Vertiefung 36 der Kapselaufnahme 26. Eine in der Kapselaufnahme 36 vorhandene Kapsel wird, dadurch beim Niederdrücken des Mundstücks 18 perforiert beziehungsweise geöffnet. Zwischen dem Grundgehäuse 14 und dem Mundstück 18 sind zwei Federelemente 42, 44 vorgesehen, die das Mundstück 18 unter Vorspannung in die Normallage beaufschlagen. Ein Niederdrücken des Mundstücks 18 erfolgt also entgegen der Vorspannkraft der beiden Federelemente 42, 44. Beim Ausführungsbeispiel gemäß den Figuren sind zwei koaxial zueinander um die Mittellängsachse 28 verlaufend angeordnete Federelemente 42, 44 in Form von Schraubenfedern vorgesehen. Aufgrund des Vorsehens dieser beiden Federelemente wird ein gleichmäßiges Beaufschlagen des Mundstücks 18 aus der Öffnungslage in die Normallage erreicht. Einem Verkanten oder Verklemmen des Mundstücks 18 während der Bewegung aus der Öffnungslage in die Normallage wird hierdurch entgegengewirkt. Das Mundstück 18 als solches wird von einem am Grundgehäuse 14 angeordneten hülsenartigen Zwischenteil 46 geführt. Das Zwischenteil 46 ist hohl ausgebildet und verschließt die Kapselaufnahme 26 zum Mundstück 18 hin mittels einer siebartigen Struktur 48. Dies hat den Vorteil, dass beim Rückführen der Nadeln 40 die gegebenenfalls von den Nadeln 40 mitgenommene Kapsel am Zwischenteil 48 beziehungsweise an der Struktur 48 abgestreift wird und zurück in die Kapselaufnahme 26 fällt. Auch beim Inhalieren durch das Mundteil 18, bei dem dann Luft aus der Kapselaufnahme 26 über das Zwischenteil 46 und das Mundstück 18 angesaugt wird, verbleibt die Kapsel in der Kapselaufnahme 26.

Um das Mundteil 18 in der Normallage entgegen der Federkraft der Federelemente 42, 44 zu sichern, sind an der siebartigen Struktur 48 des Zwischenteils 46 nach radial außen ragende Haltenasen 54 vorgesehen. Diese Haltenasen 54, die insbesondere in Figur 7 zu erkennen sind, wirken mit am Mundstück 18 vorgesehenen, nach radial innen abragenden Halteabschnitten 56 zusammen.

Um auch im eingeschwenkten Zustand des Schwenkteils 16 ein Einströmen von Luft beim Inhalieren in die Kapselaufnahme 26 zu ermöglichen, weist die Kapselaufnahme an ihrer im Wesentlichen kreisrunden Oberkante 50 einen Ansaugeinlass 52, der in Figur 5 gut zu erkennen ist, auf.

Der Gebrauchsablauf des in den Figuren dargestellten Inhalators 10 ist folgendermaßen: Zunächst wird das Schwenkteil 16 gemäß den Figuren 4 und 5 aus dem Grundgehäuse 14 herausgeschwenkt. Anschließend wird eine Trockenkapsel in die Kapselaufnahme 26 eingelegt und das Schwenkteil wird in das Grundgehäuse eingeschwenkt. Aufgrund der im eingeklappten Zustand trichterartig schräg nach unten verlaufenden Flächen 38 gelangt die Kapsel automatisch in die Vertiefung 36. Anschließend wird zum Öffnen der Kapsel das Mundstück 18 entgegen der Kraft der Federn 42, 44 nach axial unten gedrückt, wie in Figur 10 dargestellt. Die Nadeln 40 öffnen dabei die Kapsel. Beim automatischen Rückführen des Mundstücks aufgrund der Federelemente 42, 44 wird die Kapsel an der Sieb-Struktur 48 abgestreift und fällt zurück in die Kapselaufnahme 36. Anschließend kann das Pulver durch Ansetzen des Mundes an das Mundstück 18 und anschließendes Ansaugen von Luft inhaliert werden. Das in der Kapselaufnahme 26 vorhandene Pulver wird dabei mit der eingeatmeten Luft durch die Sieb-Struktur 48, die Innenseite des Zwischenteils 46 und die Innenseite des Mundstücks 18 inhaliert. Anschließend kann das Schwenkteil 16 aus dem Grundgehäuse 14 herausgeschwenkt werden und die leere Kapsel kann aus der Kapselaufnahme 26 entnommen und entsorgt werden.

Zur Lagerung von noch zu benützenden Kapseln kann die Kapselaufnahme in den Figuren nicht dargestellten Lagerraum beherbergen. Dadurch kann ein gewisser Vorrat von Kapseln im Inhalator mitgeführt werden. Der Lagerraum ist dabei insbesondere bei herausgeschwenkter Kapselaufnahme zum Einlegen bzw. Herausnehmen von zu lagernden bzw. gelagerten Kapseln zugänglich und bei eingeschwenkter Kapselaufnahme nicht zugänglich.

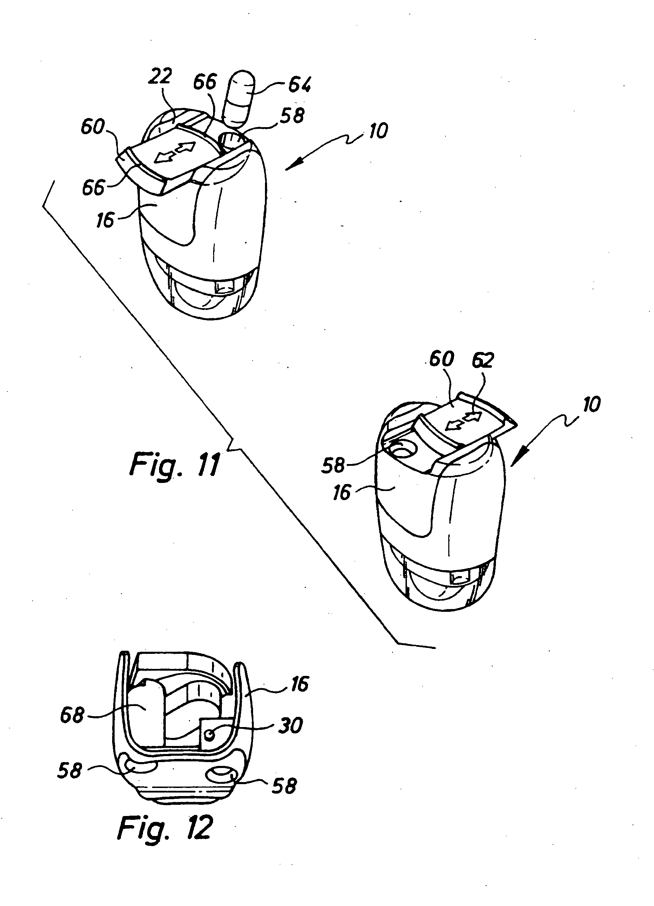

Die Figuren 11 und 12 zeigen eine Variante des Inhalators 10, der in der Figur 11 auf dem Kopf stehend dargestellt ist, bei dem das Schwenkteil 16 mit zwei zu einem Lagerraum führenden Öffnungen 58 versehen, die von einer verschieblich an der Unterseite 22 geführten Kappe 60 verschlossen sind. Die Kappe 60 kann, wie mit dem Doppelpfeil 62 dargestellt nach zwei Seiten so verschoben werden, dass jeweils eine der Öffnungen 58 freigegeben wird, so dass eine Kapsel 64 entnommen werden kann. Die Kappe 60 weist Anschläge oder Rastnoppen auf, über welche sie in der Verschlusslage, in der beide Öffnungen 58 verschlossen sind, und in den beiden Öffnungslagen gehalten wird. An der Oberseite kann die Kappe 60 mit geringfügig abragenden Stegen 66 versehen sein, die ein Verschieben der Kappe, z.B. mit dem Daumen, erleichtern.

Die Figur 12 zeigt das Schwenkteil 16 in der Gebrauchslage und mit abgezogener Kappe 60, aus dem nacheinander zwei Kapseln 64 entnommen werden können. Im Anschluss an die eine Öffnung 58 schließt sich ein Magazin 68 zur Aufnahme einer Kapsel 64 an. Es ist auch denkbar, dass im Schwenkteil 16 ein Magazin zur Aufnahme mehrerer Kaseln 64 vorgesehen ist, die dann mittels eines nicht dargestellten Spenders über eine einzige Öffnung 58 ausgegeben werden können.

## Patentansprüche

1. Trockenpulver-Inhalator (10) mit einem Grundgehäuse (14), mit einer Kapselaufnahme (26) für eine Kapsel mit Trockenpulver, mit wenigstens einem gegenüber dem Grundgehäuse (14) beweglich angeordneten, nadel- oder klingenartigen Öffnungsmittel (40) zum Öffnen der Kapsel, und mit einem Mundstück (18), durch welches das Trockenpulver einer geöffneten Kapsel inhalierbar ist, wobei das Öffnungsmittel (40) am Mundstück (18) befestigt ist und das Mundstück (18) gegenüber dem Grundgehäuse (14) aus einer Normallage in eine die Kapsel öffnende Öffnungslage bewegbar ist, **dadurch gekennzeichnet, dass** das Grundgehäuse (14) auf seiner dem Mundstück (18) abgewandten Seite ein die Kapselaufnahme (26) umfassendes oder tragendes Schwenkteil (16) vorsieht, welches gegenüber dem Grundgehäuse (14) derart um eine senkrecht zur Mittellängsachse (28) des Mundstücks (18) oder des Grundgehäuses (14) verlaufende Schwenkachse (30) schwenkbar angeordnet ist, dass es in seiner nicht ausgeschwenkten Normallage wenigstens weitgehend bündig mit der Außenseite des Grundgehäuses (14) abschließt und zum Einlegen einer Kapsel wenigstens abschnittsweise aus dem Grundgehäuse (14) herausschwenkbar ist.

2. Trockenpulver-Inhalator (10) nach Anspruch 1, **dadurch gekennzeichnet, dass** zwischen dem Grundgehäuse (14) und dem Mundstück (18) ein Federelement (42) vorgesehen ist, welches das Mundstück (18) unter Vorspannung in der Normallage hält.

3. Trockenpulver-Inhalator (10) nach Anspruch 1, **dadurch gekennzeichnet, dass** am Grundgehäuse (14) ein im Wesentlichen hülsenartiges Zwischenteil (46) zur Führung des Mundstücks (18) angeordnet ist, wobei das Zwischenteil (46) beim Inhalieren das Trockenpulver von der Kapselaufnahme (26) zum Mundstück leitet und auf der der Kapselaufnahme (26) zugewandten Seite eine siebartige Struktur (48) aufweist.

4. Trockenpulver-Inhalator (10) nach Anspruch 3, **dadurch gekennzeichnet, dass** zwischen dem Grundgehäuse (14) und dem Mundstück (18) ein Federelement (42) vorgesehen ist, welches das Mundstück (18) unter Vorspannung in der Normallage hält, wobei aufgrund des Federelements (42) das Mundstück automatisch aus der Öffnungslage in die Normallage rückgeführt und die Kapsel an der Sieb-Struktur (48) abgestreift wird, so dass die Kapsel zurück in die Kapselaufnahme (26) fällt.

5. Trockenpulver-Inhalator (10) nach einem der vorhergehenden Ansprüche, **dadurch gekennzeichnet, dass** das Öffnungsmittel (40) als wenigstens eine sich in Längsrichtung des Mundstücks (18) erstreckende Nadel ausgebildet ist.

6. Trockenpulver-Inhalator (10) nach einem der vorhergehenden Ansprüche, **dadurch gekennzeichnet, dass** die Schwenkachse (30) versetzt zur Mittellängsachse (28) des Mundstücks (18) oder des Grundgehäuses (14) angeordnet ist.

7. Trockenpulver-Inhalator (10) nach einem der vorhergehenden Ansprüche, **dadurch gekennzeichnet, dass** das Schwenkteil (16) in Seitenansicht eine U-förmige Außenkontur aufweist, die sich von der Vorderseite (20) über die Unterseite (22) bis zur Rückseite (24) des Grundgehäuses (14) erstreckt.

8. Trockenpulver-Inhalator (10) nach Anspruch 7, **dadurch gekennzeichnet, dass** zwischen den beiden U-Schenkeln des Schwenkteils (16) die Kapselaufnahme (26) angeordnet ist.

9. Trockenpulver-Inhalator (10) nach einem der vorhergehenden Ansprüche, **dadurch gekennzeichnet, dass** die Kapselaufnahme (26) eine im Wesentlichen geringfügig größer als die Kapsel ausgebildete Vertiefung (36) aufweist, an die sich trichterartig hin zur Vertiefung verlaufende Flächen (38) anschließen.

10. Trockenpulver-Inhalator (10) nach Anspruch 9, **dadurch gekennzeichnet, dass** die Längsachse der Vertiefung schräg zu einer Mittellängsebene des Grundgehäuses verläuft.

11. Trockenpulver-Inhalator (10) nach einem der vorhergehenden Ansprüche, **dadurch gekennzeichnet, dass** die Kapselaufnahme (26) durch eine im Wesentlichen kreisrunde Oberkante (50) begrenzt wird.

12. Trockenpulver-Inhalator (10) nach Anspruch 11, **dadurch gekennzeichnet, dass** im Bereich der Oberkante (50) wenigstens ein Ansaugeinlass (52) für Ansaugluft beim Inhalieren vorgesehen ist.

13. Trockenpulver-Inhalator (10) nach wenigstens einem der vorhergehenden Ansprüche, **dadurch gekennzeichnet, dass** im Schwenkteil (16) ein Lagerraum zur Bevorratung von Kapseln (64) vorgesehen ist.

## Claims

1. A dry-powder inhaler (10) having a basic housing (14), having a capsule receptacle (26) for a capsule with dry powder, having at least one needle- or blade-like opening means (40), disposed movably relative to the basic housing (14), for opening the capsule, and having a mouthpiece (18) through which the dry powder can be inhaled from an opened capsule, the opening means (40) being secured to the mouthpiece (18), and the mouthpiece (18) being movable relative to the basic housing (14) from a normal position into an opening position that opens the capsule, **characterized in that** the basic housing (14), on its side remote from the mouthpiece (18), provides a pivoting part (16), including or bearing the capsule receptacle (26), which pivoting part is disposed pivotably about a pivot axis (30), extending perpendicular to the central longitudinal axis (28) of the mouthpiece (18) or of the basic housing (14), in such a way that in its unpivoted normal position it is at least extensively flush with the outside of the basic housing (14) and can be pivoted outward in at least some portions for the insertion of a capsule.

2. The dry-powder inhaler (10) of claim 1, **characterized in that** between the basic housing (14) and the mouthpiece (18), a spring element (42) is provided, which keeps the mouthpiece (18) prestressed in the normal position.

3. The dry-powder inhaler (10) of claim 1, **characterized in that** an essentially tubular intermediate part (46) for guiding the mouthpiece (18) is disposed on the basic housing (14), and the intermediate part (46) carries the dry powder from the capsule receptacle (26) to the mouthpiece upon inhalation and has a sievelike structure (48) on its side toward the capsule receptacle (26).

4. The dry-powder inhaler (10) of claim 3, **characterized in that** between the basic housing (14) and the mouthpiece (18), a spring element (42) is provided, which keeps the mouthpiece (18) prestressed in the normal position, and because of the spring element (42), the mouthpiece is returned automatically from the opening position to the normal position and the capsule is stripped off at the sieve structure (46), so that the capsule drops back into the capsule receptacle (26).

5. The dry-powder inhaler (10) of one of the foregoing claims, **characterized in that** the opening means (40) is embodied as at least one needle extending in the longitudinal direction of the mouthpiece (18).

6. The dry-powder inhaler (10) of one of the foregoing claims, **characterized in that** the pivot axis (30) is offset from the central longitudinal axis (28) of the mouthpiece (18) or of the basic housing (14).

7. The dry-powder inhaler (10) of one of the foregoing claims, **characterized in that** the pivoting part (16), in side view, has a U-shaped outer contour which extends from the front side (20), across the underside (22,) to the back side (24) of the basic housing (14).

8. The dry-powder inhaler (10) of claim 7, **characterized in that** the capsule receptacle (26) is disposed between the two legs of the U of the pivoting part (16).

9. The dry-powder inhaler (10) of one of the foregoing claims, **characterized in that** the capsule receptacle (26) has an indentation (36), which is essentially embodied as slightly larger than the capsule and is adjoined by faces (38) extending toward the indentation in funnel-like fashion.

10. The dry-powder inhaler (10) of claim 9, **characterized in that** the longitudinal axis of the indentation extends obliquely to a central longitudinal plane of the basic housing.

11. The dry-powder inhaler (10) of one of the foregoing claims, **characterized in that** the capsule receptacle (26) is bounded by an essentially circular upper edge (50).

12. The dry-powder inhaler (10) of claim 11, **characterized in that** in the vicinity of the upper edge (50), at least one aspiration inlet (52) for aspirating air is provided.

13. The dry-powder inhaler (10) of at least one of the foregoing claims, **characterized in that** a storage chamber for keeping capsules (64) on hand is provided in the pivoting part (16).

## Revendications

1. Inhalateur à poudre sèche (10) comprenant un boîtier de base (14), comprenant un logement de capsule (26) pour une capsule logeant une poudre sèche, comprenant au moins un moyen d'ouverture (40) du type aiguille ou lame, monté mobile par rapport au boîtier de base (14), et permettant d'ouvrir la capsule, et comprenant un embout (18) par lequel la poudre sèche d'une capsule ouverte peut être inhalée, le moyen d'ouverture (40) étant fixé à l'embout (18) et l'embout (18) étant mobile par rapport au boîtier de base (14) d'une position normale à une position d'ouverture ouvrant la capsule, **caractérisé en ce que** le boîtier de base (14) présente sur sa face opposée à l'embout (18) une partie pivotante (16) entourant ou portant le logement de capsule (26), ladite partie étant disposée à l'opposé du boîtier de base (14), de manière à pouvoir pivoter autour d'un axe de pivotement (30) s'étendant perpendiculairement à l'axe longitudinal médian (28) de l'embout (18) ou du boîtier de base (14), **en ce que** l'embout, dans sa position normale non pivotée vers l'extérieur, se termine au moins dans une large mesure en affleurement avec la face extérieure du boîtier de base (14) et, pour l'insertion d'une capsule, peut être amené à pivoter à l'extérieur du boîtier de base (14) au moins par endroits.

2. Inhalateur à poudre sèche (10) selon la revendication 1, **caractérisé en ce qu'**un élément ressort (42) retenant l'embout (18) dans la position normale sous l'effet d'une précontrainte est présent entre le boîtier de base (14) et l'embout (18).

3. Inhalateur à poudre sèche (10) selon la revendication 1, **caractérisé en ce qu'**une partie intermédiaire (46) sensiblement en forme de manchon et destinée à guider l'embout (18) est disposée sur le boîtier de base (14), la partie intermédiaire (46) se déplaçant du logement de capsule (26) à l'embout lorsque la poudre sèche est inhalée et présentant sur la face tournée vers le logement de capsule (26) une structure (48) de type tamis.

4. Inhalateur à poudre sèche (10) selon la revendication 3, **caractérisé en ce qu'**un élément ressort (42) retenant l'embout (18) dans la position normale sous l'effet d'une précontrainte est présent entre le boîtier de base (14) et l'embout (18), l'embout revenant automatiquement de la position d'ouverture à la position normale sous l'action de l'élément ressort (42) et la capsule sur la structure de type tamis (48) étant retirée, de sorte que la capsule revient dans le logement de capsule (26).

5. Inhalateur à poudre sèche (10) selon l'une quelconque des revendications précédentes, **caractérisé en ce que** le moyen d'ouverture (40) est conçu comme au moins une aiguille s'étendant dans la direction longitudinale de l'embout (18).

6. Inhalateur à poudre sèche (10) selon l'une quelconque des revendications précédentes, **caractérisé en ce que** l'axe de pivotement (30) est décalé de l'axe longitudinal médian (28) de l'embout (18) ou du boîtier de base (14).

7. Inhalateur à poudre sèche (10) selon l'une quelconque des revendications précédentes, **caractérisé en ce que** la partie pivotante (16) présente en vue latérale un contour extérieur en forme de U s'étendant de la face avant (20) à la face arrière (24) du boîtier de base (14) en passant par la face inférieure (22).

8. Inhalateur à poudre sèche (10) selon la revendication 7, **caractérisé en ce que** le logement de capsule (26) est disposé entre les deux branches en U de la partie pivotante (14).

9. Inhalateur à poudre sèche (10) selon l'une quelconque des revendications précédentes, **caractérisé en ce que** le logement de capsule (26) présente un évidement (36) sensiblement légèrement plus grand que la capsule et auquel font suite les surfaces (38) s'étendant à la façon d'un entonnoir en direction de l'évidement.

10. Inhalateur à poudre sèche (10) selon la revendication 9, **caractérisé en ce que** l'axe longitudinal de l'évidement s'étend de manière oblique par rapport à un plan longitudinal médian du boîtier de base.

11. Inhalateur à poudre sèche (10) selon l'une quelconque des revendications précédentes, **caractérisé en ce que** le logement de capsule (26) est délimité par une arête supérieure (50) sensiblement circulaire.

12. Inhalateur à poudre sèche (10) selon la revendication 11, **caractérisé en ce qu'**un orifice d'aspiration (52) pour l'air aspiré lors de l'inhalation est ménagé à l'endroit de l'arête supérieure (50).

13. Inhalateur à poudre sèche (10) selon l'une quelconque des revendications précédentes, **caractérisé en ce qu'**une chambre de stockage destinée à stocker des capsules (64) est présente dans la partie pivotante (16).
